# EUROPEAN PATENT SPECIFICATION

(11) **EP 1 981 633 B1**
(45) Date of publication and mention of the grant of the patent: **05.08.2020**
(21) Application number: 07756778.2
(22) Date of filing: 08.02.2007
(51) Int. Cl.: B01J 21/04, B01J 23/68, B01J 37/08

(54) **A PROCESS FOR PREPARING A CATALYST, THE CATALYST, AND A PROCESS FOR THE PRODUCTION OF AN OLEFIN OXIDE, A 1,2-DIOL, A 1,2-DIOL ETHER, OR AN ALKANOLAMINE**
VERFAHREN ZUR HERSTELLUNG EINES KATALYSATORS, KATALYSATOR UND VERFAHREN ZUR HERSTELLUNG EINES OLEFINOXIDS, 1,2-DIOLS, 1,2-DIOLETHERS ODER ALKANOLAMINS
PROCEDE DE PREPARATION D'UN CATALYSEUR, CATALYSEUR, ET PROCEDE DE PRODUCTION D'UN OXYDE D'OLEFINE, D'UN 1,2-DIOL, D'UN ETHER DE 1,2-DIOL, OU D'UNE ALCANOLAMINE

(30) Priority: 10.02.2006 US 772420 P
(43) Date of publication of application: 22.10.2008
(73) Proprietor: Shell Internationale Research Maatschappij B.V., 2596 HR The Hague (NL)
(72) Inventor: REMUS, Donald James, Stow, OH 44224 (US); RICHARD, Michael Alan, Fulshear, Texas 77441 (US); SZYMANSKI, Thomas, Hudson, OH 44236 (US)
(74) Representative: Shell Legal Services IP
(86) International application number: PCT/US2007/061861
(87) International publication number: WO 2007/095453

(56) References cited:
- EP-A1- 0 496 470
- WO-A-96/23585
- US-A- 5 380 697

## Description

### Field of the Invention

The present invention relates to a process for preparing an epoxidation catalyst.

### Background of the Invention

In olefin epoxidation, a feed containing an olefin and an oxygen source is contacted with a catalyst under epoxidation conditions. The olefin is reacted with oxygen to form an olefin oxide. A product mix results that contains olefin oxide and, typically, unreacted feed and combustion products.

The olefin oxide may be reacted with water to form a 1,2-diol, with an alcohol to form a 1,2-diol ether, or with an amine to form an alkanolamine. Thus, 1,2-diols, 1,2-diol ethers, and alkanolamines may be produced in a multi-step process initially comprising olefin epoxidation and then the conversion of the formed olefin oxide with water, an alcohol, or an amine.

Olefin epoxidation catalysts comprise a silver component, usually with one or more additional elements deposited therewith, on a carrier. The silver and additional elements may be deposited by using one or more impregnation solutions. The carrier is typically formed of a refractory material, such as alpha alumina. To be commercially viable, the carrier may typically possess pores through which the catalytic component may be deposited. Pores may be incorporated into the carrier through the use of burnout materials. Typically, the burnout materials are naturally occurring organic materials such as shells obtained from a tree or synthetic organic materials such as polymers.

The materials used for making the carriers may include: a sinterable material; a thermally decomposable material, i.e., the burnout material; a diluent; and lubricants or other processing aids. Carriers are manufactured by selecting the raw materials; processing the raw materials to produce unfired carrier bodies, also known as greenware; and sintering the unfired carrier bodies to produce the carrier.

US-5801259 discloses an ethylene oxide catalyst comprising a carrier prepared without using a burnout material. The use of a burnout material is avoided because burnout materials tend to leave metallic oxide leachables in the carrier after the burnout has been removed. Some leachables, and particularly some metallic leachables, are undesirable because they are known to adversely affect the stability of the selectivity and/or the activity of the catalyst.

The catalyst performance may be assessed on the basis of selectivity, activity and stability of operation. The selectivity is the fraction of the converted olefin yielding the desired olefin oxide. As the catalyst ages, the fraction of the olefin converted normally decreases with time and to maintain a constant level of olefin oxide production the temperature of the reaction may be increased. However this adversely affects the selectivity of the conversion to the desired olefin oxide. In addition, the equipment used can tolerate temperatures only up to a certain level so that it is necessary to terminate the reaction when the reaction temperature would reach a level inappropriate for the reactor. Thus the longer the activity and selectivity can be maintained at acceptable values, the longer the catalyst charge can be kept in the reactor and the more product is obtained. Quite modest improvements in the selectivity and maintenance of the selectivity and activity over long periods yield substantial dividends in terms of process efficiency.

### Summary of the Invention

The present invention provides a process for preparing a catalyst as defined in the claims.

### Detailed Description of the Invention

Depending upon the source, thermally decomposable materials may widely vary in their content of impurities. It has been found that the existence of variable quantities of potassium-containing compounds in the thermally decomposable material can significantly and adversely impact the manufacture of the carrier, the manufacture of the catalyst and the performance of the catalyst. Variability in the quantities of potassium-containing compounds can cause problems in the production of the carrier, for example batch-to-batch inconsistencies in the carrier.

During catalyst manufacture, the metal deposition process can be adversely affected by the presence of variable quantities of potassium-containing compounds left in the pores from the removal of the thermally decomposable material. Without the intention of suggesting this invention is limited by one theory or mechanism, this may lead to dissolution of these leftover potassium-containing compounds in the impregnation solution. Moreover, as suggested in US 5187140, the impregnation solution may be reused several times in catalyst manufacture. When the impregnation solution is reused, the leftover potassium-containing compounds may build up in the reused impregnation solution and be deposited in varying amounts on subsequent batches of carrier.

Treating the thermally decomposable material to reduce the concentration of potassium-containing compounds prior to forming the carrier, in accordance with this invention, improves the preparation of the carrier, in particular improves the consistency of the carrier. Also, treating the thermally decomposable material in accordance with this invention improves the manufacture of the catalyst, in particular improves the consistency of the catalyst performance.

Moreover, when a catalyst is prepared in accordance with this invention, an advantage is achieved in the performance of the catalyst as compared to the performance of a catalyst using a carrier prepared without treating the thermally decomposable material, i.e., burnout material. The advantage may be found, for example, in an improved selectivity of the catalyst and an improved resistance against performance decline, in particular activity and selectivity.

A thermally decomposable material may function as a pore former. As used herein, the thermally decomposable material is a solid in particulate form. The thermally decomposable material is mixed with a heat sinterable material prior to the heating step. Individual particles of thermally decomposable material occupy a multitude of small spaces in the mixture. The individual particles of thermally decomposable material are removed by thermal decomposition during the heating step thereby leaving pores in the ceramic material forming the carrier. The pores may also be described as a plurality of voids distributed throughout the carrier. The thermally decomposable material should not be soluble in any of the other ingredients used to make the carrier. Similarly, the thermally decomposable material should not dissolve any of the other ingredients. Because the thermally decomposable material occupies a volume prior to the heating step and the spaces occupied by the material remain generally unoccupied after the heating step has been completed, the material functions as a pore former.

The thermally decomposable material useful in a process of this invention is an organic material. Suitably the chemical formula of the organic material comprises carbon and hydrogen. The thermally decomposable material may be a synthetic or a naturally occurring material or a mixture of the same. The thermally decomposable material may be an organic material that has a decomposition temperature which is no greater than the sintering temperature of the heat sinterable material. This insures that the thermally decomposable material is at least partly removed prior to or simultaneously with the sintering of the heat sinterable material. To facilitate decomposition, the chemical formula of the thermally decomposable material may preferably comprise carbon, hydrogen and oxygen. The decomposition temperature may be lowered by the presence of oxygen.

After thermal decomposition, the volume of the solid thermally decomposable material may be reduced by at least 98%, preferably at least 99%, relative to the pre-decomposition volume of the thermally decomposable material. This reduction in volume is achieved by converting the thermally decomposable material into one or more gaseous byproducts. Another byproduct of thermal decomposition of the thermally decomposable material may be ash. The term "ash" as used herein is the residue left after decomposing the thermally decomposable material. The quantity of ash may be less than 2 percent by weight, preferably less than one percent by weight, relative to the weight of the thermally decomposable material.

The thermally decomposable material may be a synthetic material. The synthetic material may be a polymer material. The polymer material may be formed using an emulsion polymerization, including suspension polymerization, which is often preferred since the polymer can be obtained in the form of fine particles that are directly usable as thermally decomposable material. Preferably, the polymer material may be formed using anionic polymerization. The polymer material may be olefin polymers and copolymers, for example polyethylene, polypropylene, polystyrene, polyvinyl alcohol, ethylene-vinyl acetate and ethylene-vinyl alcohol copolymers; diene polymers and copolymers such as polybutadiene, EPDM rubber, styrene-butadiene copolymers and butadiene-acrylonitrile rubbers; polyamides such as polyamide-6, and polyamide-66; polyesters such as polyethylene terephthalate. Preferably, the polymer material may be hydrocarbon polymers such as polyolefins, more preferably polypropylene. The thermally decomposable material may be a naturally occurring organic material. Suitable naturally occurring organic materials may be obtained from a portion of a plant or tree, for example sawdust. Naturally occurring organic materials may also be derived from a plant or tree, for example charcoal. Naturally occurring organic materials may be obtained from a portion of a fruit from a plant or tree. For example, naturally occurring organic materials may include pecan shells, almond shells, cashew shells, walnut shells, olive pits, apricot pits, peach pits and corn cobs that may have been ground or milled into a particulate form commonly known as a powder.

While most thermally decomposable materials may use a single source of raw materials, thermally decomposable materials may be formed by mixing different sources of raw materials, such as a combination of almond shells, which have a first chemical composition, and peach pits, which have a second chemical composition.

The thermally decomposable material may be screened or otherwise sorted to limit the size of the individual particles to a specific particle size range. If desired, a first thermally decomposable material, having particles within a first particle size range, may be combined with a second thermally decomposable material, having particles within a second particle size range, to obtain a multimodal distribution of pore sizes in the porous ceramic material of the carrier. The limitations on a particle size range are determined by the size of the pores to be created in the porous ceramic material of the carrier.

The thermally decomposable material has a volume average particle size of at most 1000 microns, preferably at most 500 microns, more preferably at most 400 microns. The thermally decomposable material has a volume average particle size of at least 1 micron, preferably at least 5 microns, more preferably at least 10 microns. The thermally decomposable material may have a volume average particle size in the range of from 5 to 500 microns, preferably from 10 to 400 microns, more preferably from 15 to 300.

As used herein, the volume average particle size represents a particle diameter at which there are equal spherical equivalent volumes of particles larger and particles smaller than the stated average particle size.

The volume average particle size can be measured by a laser light scattering instrument, for example, a Horiba LA900 particle size analyzer. The method includes dispersing the particles by ultrasonic treatment, thus breaking up secondary particles, if any, into primary particles. This sonification treatment is continued until no further change in value is noticed, which typically requires a 5 minute sonification when using the Horiba LA900 particle size analyzer.

The thermally decomposable materials generally contain potassium, and/or compounds containing potassium, which have been found to be detrimental to carrier and catalyst manufacture and performance of catalysts. Furthermore, the concentrations of potassium found in commercially available naturally occurring organic materials may be highly variable. The concentration of potassium in the naturally occurring organic materials may be impacted by several factors including the soil conditions in which the tree or plant was grown as well as the equipment and storage conditions used to grind and store the naturally occurring organic materials. Without practicing the present invention, many naturally occurring organic materials are less suitable for the production of catalyst carriers that must provide catalysts with consistent superior performance, for example, in an epoxidation reaction. This is due to the variability in the concentration of potassium.

To address the problems caused by the presence of potassium in the thermally decomposable material, the thermally decomposable material may be treated to reduce a first concentration of potassium in the thermally decomposable material to yield a treated thermally decomposable material, the treated thermally decomposable material comprising a second concentration of potassium. The term "first concentration of potassium", as used herein, is the concentration of the potassium in the thermally decomposable material prior to treating, measured as the weight of potassium (as the element) relative to the weight of the thermally decomposable material. The term "second concentration of potassium", as used herein, is the concentration of potassium in the treated thermally decomposable material, measured as the weight of potassium (as the element) relative to the weight of the treated thermally decomposable material. Potassium, as used herein, is meant to include potassium in any form such as an ion, compound, complex, or species.

The total quantity of potassium in the thermally decomposable material is reduced by the treatment by at least 30 percent, most preferably by at least 40 percent, in particular by at least 50 percent, and more in particular by at least 60 percent, calculated based on the weight of the potassium in the treated thermally decomposable material relative to the weight of potassium in the untreated thermally decomposable material.

The first concentration of potassium may be at most 25000, typically at most 10000, more typically at most 5000, most typically at most 2000 parts by million weight (ppmw), relative to the weight of the thermally decomposable material. The first concentration of potassium may be at least 1, typically at least 25, more typically at least 50 ppmw, relative to the weight of the thermally decomposable material.

The second concentration of potassium may be at most 22000, preferably at most 15000, more preferably at most 8000, most preferably at most 1000 ppmw, relative to the dry weight of the treated thermally decomposable material. The second concentration of potassium may be at least 0.5, or at least 10, or at least 15 ppmw, relative to the dry weight of the treated thermally decomposable material.

As used herein, the concentration of an alkali metal, in particular potassium, in the thermally decomposable material is determined by drying the thermally decomposable material at 110 °C until a constant weight ("dry weight") is achieved; combusting the thermally decomposable material, thereby generating ash and gaseous byproducts; and then using an inductively coupled plasma technique (ICP) to determine the quantity of alkali metals in the ash. The quantity of nitric acid extractable compounds, for example an alkali metal, in a carrier is determined by boiling a standard amount of the carrier in a standard volume of 10% nitric acid for 15 minutes, which extracts the metal in the form of the soluble nitrate, and then analyzing for the residual metallic value. The quantity of the alkali metal, such as potassium, is reported as a concentration (ppmw) based on the dry weight of the thermally decomposable material or the weight of the carrier.

The treatment may involve the use of a fluid. The fluid used in the treatment process may be any fluid that is able to remove potassium ions and/or compounds that contain the potassium from the thermally decomposable material. The treatment fluid may be a liquid, a supercritical fluid or a gas. Preferably, the treatment fluid is a liquid, more preferably an aqueous liquid, most preferably deionized water. The treatment fluid may be an aqueous liquid further comprising one or more inorganic or organic compounds, such compounds may be alcohols, for example methanol or ethanol; ketones, for example acetone or methyl ethyl ketone; or acids, for example acetic acid, citric acid, oxalic acid, or ammonium salts. The aqueous liquid may additionally comprise one or more additives such as a chelating agent and/or wetting agent. The ability of the aqueous liquid to remove potassium from the thermally decomposable material may be improved with the addition of additives to the liquid. The chelating agents may be, for example, ethylenediaminetetraacetic acid (EDTA), nitrilotriacetic acid, alkanolamines, or bases. The wetting agent may be, for example, nonionic surfactants.

The thermally decomposable material may be treated by washing, soaking, spraying, extracting, or supercritical fluid extraction, or otherwise contacting the thermally decomposable material with the treatment fluid for a sufficient period of time to remove the potassium ions and/or compounds.

In embodiments where the treatment fluid is a liquid, the temperature of the treatment fluid may be any temperature where the treatment fluid at least in part remains in the liquid form. Suitably, the treatment fluid may be in the range of from 0 to 200 °C, in particular from 10 to 50 °C, more in particular from 15 to 40 °C. The treatment fluid may be contacted with the thermally decomposable material at any pressure. Suitably, the pressure may be in the range of from 0.5 to 10 bar, in particular from 1 to 5 bar, more in particular about 1 bar (atmospheric pressure).

In embodiments where the treatment fluid is not a liquid, for example a gas or vapor, the temperature of the treatment fluid may be in the range of from 15 to 400 °C, in particular from 20 to 350 °C, more in particular from 25 to 300 °C. The treatment fluid may be contacted with the thermally decomposable material at a pressure in the range of from 1 to 500 bar, in particular from 5 to 400 bar.

The treatment fluid may then be removed from the thermally decomposable material to yield a treated thermally decomposable material having a second concentration of potassium which is lower than the first concentration of potassium. After the treatment fluid is removed, the thermally decomposable material may preferably be dried. Drying may be performed by a mechanical process, for example tumbling. Drying may be performed by a thermal process, for example heating between 20 and 200 °C and preferably between 30 and 150 °C. Preferably, drying is performed to the extent that the treated thermally decomposable material reaches its dry weight, as defined hereinbefore, or that contains in addition less than 8 %wt. of fluid, more preferably less than 5 % wt., most preferably less than 3 % wt.

The method for measuring the moisture content of the treated thermally decomposable material can be determined by drying the treated thermally decomposable material at 110 °C until a constant weight is achieved and calculating the percentage of weight loss in the treated thermally decomposable material.

The thermally decomposable material may be treated in two or more steps with a suitable treatment fluid. One or more treatment fluids may be used to remove potassium from the thermally decomposable material. For example, in a first treatment step, the thermally decomposable material contacts a first treatment fluid. In a second treatment step, the thermally decomposable material contacts a second treatment fluid. The chemical compositions of the first and second treatment fluids may be the same or different.

Use of multiple steps in the treatment with the same treatment fluid may depend upon the quantity of potassium to be removed, the solubility of potassium in the treatment fluid, and the fluid treatment ratio. The use of multiple steps in the treatment with different treatment fluids may be used when the thermally decomposable material contains two or more chemical species that may not be soluble in the same treatment fluid. For example, in a first treatment step, the first treatment fluid, having a first chemical composition, may be contacted with the thermally decomposable material to remove one or more of the chemical species. In a second treatment step, the second treatment fluid, having a second chemical composition different from the first treatment fluid, may be contacted with the thermally decomposable material to remove the one or more different chemical species than the first treatment fluid.

The weight ratio of treatment fluid to thermally decomposable material for use in a treatment step may be in the range of from 1:1 to 25:1, preferably from 2:1 to 15:1. For example, ratios may include 9:1, 6:1, 4:1, and 2:1. Lower ratios of treatment fluid to thermally decomposable material may limit the cost and time involved in reducing the concentration of potassium.

If desired, the treated thermally decomposable material may be combined in the mixture with an untreated thermally decomposable material. Typically, the weight fraction of the treated thermally decomposable material is at most 100%, more typically at most 99 %, most typically at most 98 %, in particular at most 95 %, relative to the total weight of the thermally decomposable material. Typically, the weight fraction of the treated thermally decomposable material is at least 5 %, more typically at least 10 %, most typically at least 20 %, in particular at least 25 %, relative to the total weight of the thermally decomposable material.

A mixture may be prepared by mixing a heat sinterable material with a treated thermally decomposable material, as described above. The heat sinterable materials may be an alumina, silica, titania, silicon carbide, silicon nitride, niobia, magnesia, zinc oxide, zirconia based compounds, or combinations thereof. Preferably, the heat sinterable material comprises alpha alumina. The mixture may additionally comprise minor amounts of other ceramic oxides and/or alkaline earth metal compounds and alkali metal compounds.

The heat sinterable material may have a volume average particle size of from 0.5 to 100 microns, preferably from 1 to 80 microns. The heat sinterable material may have an average crystallite size from 0.1 to 5 microns, preferably from 1 to 4 microns. The average crystallite size can be determined by measuring the maximum dimension of a number of crystallites and taking the average thereof.

In an embodiment, the heat sinterable material comprises alpha alumina and zirconia. Zirconia may be present in a quantity of at most 15 % wt., preferably at most 10 % wt., more preferably at most 5 % wt., relative to the total weight of the carrier. Zirconia may be present in a quantity of at least 0.01 % wt., preferably at least 0.05 % wt., more preferably at least 0.5 % wt., relative to the total weight of the carrier. Preferably, zirconia may be present in a quantity in the range of from 0.05 to 2 % wt., relative to the total weight of the carrier.

It is also a common expedient to use a bond material, i.e. a material which reduces the length of sintering time applied to bond the particles together, and it is preferred to employ such bond material when practicing this invention. It is advantageous that the bond material also forms a coating on at least a part of the carrier surface, which makes the carrier surface more receptive. The bond material may be based on a silica-containing composition comprising a crystallization inhibitor, inhibiting the formation of crystalline silica-containing compositions. It is also preferred that the bond material provides a coating of a non-crystalline silica compound to the carrier surface.

In an embodiment, silica-containing compositions for use as a bond material comprise an amorphous silica compound which may be, for example, a silica sol, a precipitated silica, an amorphous silica, or an amorphous sodium or lithium silicate.

In another embodiment, silica-containing compositions for use as a bond material may comprise an alkaline earth metal silicate. The alkaline earth metal silicate may be magnesium silicate or calcium silicate, preferably magnesium silicate. Reference may be made, for example, to US-5100859.

Typically, silica-containing compositions for use as a bond material may also comprise hydrated alumina. The preferred alumina hydrate is boehmite, though gibbsite, bayerite or diaspore may also be used.

A convenient bond material may comprise a mixture of boehmite, ammonium or alkaline earth metal silicate or silica sol, and a water soluble sodium salt. Similar effects can be achieved by incorporation of conventional ceramic bonds formulated to contain aluminosilicates and a sodium or lithium component.

The mixture may contain a quantity of thermally decomposable material of at most 40 %w, preferably at most 30 %w, more preferably at most 25 %w, relative to the total weight of the mixture. The mixture may contain a quantity of thermally decomposable material of at least 2 %w, preferably at least 5 %w, more preferably at least 10 %w, relative to the total weight of the mixture. Preferably, the mixture may contain a quantity of thermally decomposable material in the range of from 2 to 40 %w, more preferably in the range of from 5 to 30 %w, relatively to the total weight of the mixture. The mixture may contain a quantity of heat sinterable material of at most 98 %w, preferably at most 75 %w, more preferably at most 70 %w, relative to the total weight of the mixture.

The mixture may be formed into carrier bodies. In general, the size of the carrier bodies is determined by the dimensions of the reactor in which they are to be deposited. Generally, however, it is found very convenient to use carrier bodies in the form of powdery particles, trapezoidal bodies, cylinders, saddles, spheres, doughnuts, and the like. The cylinders may be solid or hollow, straight or bent, and they may have their length from 4 to 20 mm, typically from 5 to 15 mm; their outside diameter from 4 to 20 mm, typically from 5 to 15 mm; and their inside diameter from 0.1 to 6 mm, typically from 0.2 to 4 mm. The cylinders may have a ratio of length to outside diameter in the range of from 0.5 to 2, typically from 0.8 to 1.2.

The carrier bodies may be formed from the mixture by any convenient molding process, such as spraying, spray drying, agglomeration or pressing, and preferably they are formed by extrusion of the mixture. For applicable methods, reference may be made to, for example, US-A-5145824, US-A-5512530, US-A-5384302, US-A-5100859 and US-A-5733842. To facilitate such molding processes, in particular extrusion, the mixture may suitably be compounded with up to about 30 %w and preferably from 2 to 25 %w, based on the weight of the mixture, of processing aids. Processing aids (also referred to by the term "extrusion aids") are known in the art (cf., for example, "Kirk-Othmer Encyclopedia of Chemical Technology", 4th edition, Volume 5, pp. 610 ff.). Suitable processing aids are typically liquids or greasy substances, for example petroleum jelly, hydrogenated oil, synthetic alcohol, synthetic ester, glycol, or polyolefin oxide. Boric acid may also be added to the mixture, for example in a quantity of up to 0.5 %w, more typically in a quantity of from 0.01 to 0.5 %w. The effect of the presence of boric acid may be a further reduced content of leachable alkali metal ions in the carrier after firing. Enough water may be added to the mixture to make the mixture moldable (by the term "the weight of the mixture", as used hereinbefore, is meant the weight of the total mixture, but excluding the weight of any added water).

The formed carrier bodies may be dried to remove at least a portion of the water present, if any. Water might convert to steam during the heating step, described hereinafter, and adversely affect the physical integrity of the shaped bodies. The drying may occur after the preparation of the mixture and optional forming of the mixture into a plurality of shaped bodies. The drying step may be combined with the heating step by controlling the thermal profile of the oven or kiln. Drying may take place between 20 and 400 °C and preferably between 30 and 300 °C, typically for a period of up to 100 hours and preferably for from 5 minutes to 50 hours. Typically, drying is performed to the extent that the mixture contains less than 2 %w of water.

The heating step may be carried out in any atmosphere, such as in air, nitrogen, argon, or helium, or mixtures thereof. Preferably, the heating step is at least in part or entirely carried out in an oxidizing atmosphere, such as an oxygen containing atmosphere. In the presence of such oxidizing atmosphere at least part of the thermally decomposable material is removed by combustion. The temperature of the heating step may be between 1150 and 1650 °C, typically between 1300 and 1530 °C, preferably between 1300 and 1520 °C, typically for a period of up to about 8 hours and preferably for from 2 to 6 hours. During the heating step, the thermally decomposable material is decomposed and the heat sinterable material in the mixture is sintered thereby forming the carrier.

As used herein, sintering means the process of firing and consolidating a body from powder particles. The particles are bound to adjoining particles. Voids may exist between and/or within the particles.

The carrier for use in this invention has typically a content of nitric acid extractable components (as the weight of the metal, or SiO₂), relative to the weight of the carrier as follows:
sodium: less than 2000 ppmw, preferably less than 1500 ppmw, and/or
calcium: less than 800 ppmw, preferably less than 600 ppmw, and/or
magnesium: less than 1000 ppmw, preferably less than 800 ppmw, and/or
aluminum: less than 1100 ppmw, preferably less than 800 ppmw, and/or
silicate: less than 2000 ppmw, preferably less than 1500 ppmw.

The quantity of potassium in the carrier may vary depending on many factors, in particular the quantity of thermally decomposable material used and the second concentration of potassium in the treated thermally decomposable material. Preferably, the quantity of potassium in the carrier may be at most 200, more preferably at most 150, most preferably at most 100 ppmw, relative to the total weight of the carrier. Typically, the quantity of potassium in the carrier may be at least 1, more typically at least 5 ppmw, relative to the total weight of the carrier.

The time and temperature applied during the heating step affects the morphology of the carrier for a given mixture, for example surface area, water absorption, total pore volume, and pore volume distribution.

The surface area of the carrier may suitably be at least 0.1 m²/g, preferably at least 0.3 m²/g, more preferably at least 0.5 m²/g, and in particular at least 0.6 m²/g, relative to the weight of the carrier; and the surface area may suitably be at most 10 m²/g, preferably at most 6 m²/g, and in particular at most 4 m²/g, relative to the weight of the carrier. "Surface area" as used herein is understood to relate to the surface area as determined by the B.E.T. (Brunauer, Emmett and Teller) method as described in Journal of the American Chemical Society 60 (1938) pp. 309-316. High surface area carriers, in particular when they are alpha alumina carriers optionally comprising in addition silica, alkali metal and/or alkaline earth metal components, provide improved performance and stability of operation.

The water absorption of the carrier may suitably be at least 0.2 g/g, preferably at least 0.25 g/g, more preferably at least 0.3 g/g, most preferably at least 0.35 g/g; and the water absorption may suitably be at most 0.85 g/g, preferably at most 0.7 g/g, more preferably at most 0.65 g/g, most preferably at most 0.6 g/g. The water absorption of the carrier may be in the range of from 0.2 to 0.85 g/g, preferably in the range of from 0.25 to 0.7 g/g, more preferably from 0.3 to 0.65 g/g, most preferably from 0.3 to 0.6 g/g. A higher water absorption may be in favor in view of a more efficient deposition of the metal and further elements, if any, on the carrier by impregnation. However, at a higher water absorption, the carrier, or the catalyst made therefrom, may have lower crush strength. As used herein, water absorption is deemed to have been measured in accordance with ASTM C20, and water absorption is expressed as the weight of the water that can be absorbed into the pores of the carrier, relative to the weight of the carrier.

The catalyst may comprise a metal. Preferably, the metal may be a catalytically active metallic species. The catalytically active metallic species may comprise one or more of silver, molybdenum, nickel, and tungsten, or compounds thereof, preferably silver.

The following description provides details of a silver catalyst, its preparation and its use in an epoxidation process.

The preparation of the silver catalyst is known in the art and the known methods are applicable to the preparation of the catalyst which may be used in the practice of this invention. Methods of depositing silver on the carrier include impregnating the carrier or carrier bodies with a silver compound containing cationic silver and performing a reduction to form metallic silver particles. Reference may be made, for example, to US-A-5380697, US-A-5739075, EP-A-266015, and US-B-6368998.

The reduction of cationic silver to metallic silver may be accomplished during a step in which the catalyst is dried, so that the reduction as such does not require a separate process step. This may be the case if the silver containing impregnation solution comprises a reducing agent, for example, an oxalate, a lactate or formaldehyde.

Appreciable catalytic activity is obtained by employing a silver content of the catalyst of at least 10 g/kg, relative to the weight of the catalyst. Preferably, the catalyst comprises silver in a quantity of from 50 to 500 g/kg, more preferably from 100 to 400 g/kg.

The silver catalyst may additionally comprise a promoter component. The promoter component may be one or more of rhenium, tungsten, molybdenum, chromium, and mixtures thereof. Preferably, the promoter component comprises, as an element, rhenium.

The promoter component may preferably be present in a quantity of at least 0.01 mmole/kg, more preferably at least 0.1 mmole/kg, and most preferably at least 0.5 mmole/kg, calculated as the total quantity of the element (that is rhenium, tungsten, molybdenum and/or chromium) relative to the weight of the catalyst. The promoter component may be present in a quantity of at most 50 mmole/kg, preferably at most 10 mmole/kg, more preferably at most 5 mmole/kg, calculated as the total quantity of the element relative to the weight of the catalyst. The form in which the promoter component may be deposited onto the carrier is not material to the invention. For example, the promoter component may suitably be provided as an oxide or as an oxyanion, for example, as a rhenate, perrhenate, or tungstate, in salt or acid form.

When the silver catalyst comprises a rhenium containing promoter component, rhenium may typically be present in a quantity of at least 0.1 mmole/kg, more typically at least 0.5 mmole/kg, and preferably at least 1.0 mmole/kg, in particular at least 1.5 mmole/kg, calculated as the quantity of the element relative to the weight of the catalyst. Rhenium is typically present in a quantity of at most 5 mmole/kg, preferably at most 3 mmole/kg, more preferably at most 2 mmole/kg, in particular at most 1.5 mmole/kg.

Further, when the silver catalyst comprises a rhenium containing promoter component, the catalyst may preferably comprise a rhenium copromoter, as a further component deposited on the carrier. Suitably, the rhenium copromoter may be one or more elements of tungsten, chromium, molybdenum, sulfur, phosphorus, boron, and mixtures thereof. Preferably, the rhenium copromoter may be one or more of tungsten, chromium, molybdenum, sulfur, and mixtures thereof. It is particularly preferred that the rhenium copromoter comprises, as an element, tungsten.

The rhenium copromoter may preferably be present in a total quantity of at least 0.01 mmole/kg, more preferably at least 0.1 mmole/kg, and most preferably at least 0.5 mmole/kg, calculated as the element (i.e. the total of tungsten, chromium, molybdenum, sulfur, phosphorus and/or boron), relative to the weight of the catalyst. The rhenium copromoter may be present in a total quantity of at most 40 mmole/kg, preferably at most 20 mmole/kg, more preferably at most 10 mmole/kg, most preferably at most 5 mmole/kg, on the same basis. The form in which the rhenium copromoter may be deposited on the carrier is not material to the invention. For example, it may suitably be provided as an oxide or as an oxyanion, for example, as a sulfate, borate or molybdate, in salt or acid form.

The catalyst preferably comprises silver, the promoter component, and a component comprising a further element, deposited on the carrier. Eligible further elements may be one or more of nitrogen, fluorine, alkali metals, alkaline earth metals, titanium, hafnium, zirconium, vanadium, thallium, thorium, tantalum, niobium, gallium and germanium and mixtures thereof. Preferably, the alkali metals are selected from lithium, potassium, rubidium and cesium. Most preferably, the alkali metal is lithium, potassium and/or cesium. Preferably, the alkaline earth metals are selected from calcium and barium. Typically, the further element is present in the catalyst in a total quantity of from 0.01 to 500 mmole/kg, more typically from 0.05 to 100 mmole/kg, calculated as the element on the weight of the catalyst. The further elements may be provided in any form. For example, salts of an alkali metal or an alkaline earth metal are suitable. For example, salts of lithium may be lithium hydroxide or lithium nitrate.

Preferred amounts of the components of the catalysts are, when calculated as the element, relative to the weight of the catalyst:
- silver from 10 to 500 g/kg,
- rhenium from 0.01 to 50 mmole/kg, if present,
- the further element or elements, if present, each from 0.1 to 500 mmole/kg, and,
- the rhenium co-promoter from 0.1 to 30 mmole/kg, if present.

As used herein, the quantity of alkali metal present in the catalyst is deemed to be the quantity insofar as it can be extracted from the catalyst with de-ionized water at 100 °C. The extraction method involves extracting a 10-gram sample of the catalyst three times by heating it in 20 ml portions of de-ionized water for 5 minutes at 100 °C and determining in the combined extracts the relevant metals by using a known method, for example atomic absorption spectroscopy.

As used herein, the quantity of alkaline earth metal present in the catalyst is deemed to be the quantity insofar as it can be extracted from the catalyst with 10 %w nitric acid in de-ionized water at 100 °C. The extraction method involves extracting a 10-gram sample of the catalyst by boiling it with a 100 ml portion of 10 %w nitric acid for 30 minutes (1 atm., i.e. 101.3 kPa) and determining in the combined extracts the relevant metals by using a known method, for example atomic absorption spectroscopy. Reference is made to US-A-5801259.

Although the epoxidation process may be carried out in many ways, it is preferred to carry it out as a gas phase process, i.e. a process in which the feed is contacted in the gas phase with the catalyst which is present as a solid material, typically in a packed bed. Generally the process is carried out as a continuous process.

The olefin for use in the epoxidation process may be any olefin, such as an aromatic olefin, for example styrene, or a di-olefin, whether conjugated or not, for example 1,9-decadiene or 1,3-butadiene. Typically, the olefin is a monoolefin, for example 2-butene or isobutene. Preferably, the olefin is a mono-α-olefin, for example 1-butene or propylene. The most preferred olefin is ethylene.

The olefin concentration in the feed may be selected within a wide range. Typically, the olefin concentration in the feed will be at most 80 mole-%, relative to the total feed. Preferably, it will be in the range of from 0.5 to 70 mole-%, in particular from 1 to 60 mole-%, on the same basis. As used herein, the feed is considered to be the composition which is contacted with the catalyst.

The epoxidation process may be air-based or oxygen-based, see "Kirk-Othmer Encyclopedia of Chemical Technology", 3rd edition, Volume 9, 1980, pp. 445-447. In the air-based process air or air enriched with oxygen is employed as the source of the oxidizing agent while in the oxygen-based processes high-purity (at least 95 mole-%) oxygen is employed as the source of the oxidizing agent. Presently most epoxidation plants are oxygen-based.

The oxygen concentration in the feed may be selected within a wide range. However, in practice, oxygen is generally applied at a concentration which avoids the flammable regime. Typically, the concentration of oxygen applied will be within the range of from 1 to 15 mole-%, more typically from 2 to 12 mole-% of the total feed.

In order to remain outside the flammable regime, the concentration of oxygen in the feed may be lowered as the concentration of the olefin is increased. The actual safe operating ranges depend, along with the feed composition, also on the reaction conditions such as the reaction temperature and the pressure.

A reaction modifier may be present in the feed for increasing the selectively, suppressing the undesirable oxidation of olefin or olefin oxide to carbon dioxide and water, relative to the desired formation of olefin oxide. Many organic compounds, especially organic halides and organic nitrogen compounds, may be employed as the reaction modifier. Nitrogen oxides, hydrazine, hydroxylamine or ammonia may be employed as well. It is frequently considered that under the operating conditions of olefin epoxidation the nitrogen containing reaction modifiers are precursors of nitrates or nitrites, i.e. they are so-called nitrate- or nitrite-forming compounds (cf. e.g. EP-A-3642 and US-A-4822900).

Organic halides are the preferred reaction modifiers, in particular organic bromides, and more in particular organic chlorides. Preferred organic halides are chlorohydrocarbons or bromohydrocarbons. More preferably they are selected from the group of methyl chloride, ethyl chloride, ethylene dichloride, ethylene dibromide, vinyl chloride or a mixture thereof. Most preferred reaction modifiers are ethyl chloride and ethylene dichloride.

Suitable nitrogen oxides are of the general formula NOₓ wherein x is in the range of from 1 to 2, and include for example NO, N₂O₃ and N₂O₄. Suitable organic nitrogen compounds are nitro compounds, nitroso compounds, amines, nitrates and nitrites, for example nitromethane, 1-nitropropane or 2-nitropropane. In preferred embodiments, nitrate- or nitrite-forming compounds, e.g. nitrogen oxides and/or organic nitrogen compounds, are used together with an organic halide, in particular an organic chloride.

The reaction modifiers are generally effective when used in low concentration in the feed, for example up to 0.1 mole-%, relative to the total feed, for example from 0.01×10⁻⁴ to 0.01 mole-%. In particular when the olefin is ethylene, it is preferred that the reaction modifier is present in the feed at a concentration of from 0.1×10⁻⁴ to 50×10⁻⁴ mole-%, in particular from 0.3×10⁻⁴ to 30×10⁻⁴ mole-%, relative to the total feed.

In addition to the olefin, oxygen and the reaction modifier, the feed may contain one or more optional components, such as carbon dioxide, inert gases and saturated hydrocarbons. Carbon dioxide is a by-product in the epoxidation process. However, carbon dioxide generally has an adverse effect on the catalyst activity. Typically, a concentration of carbon dioxide in the feed in excess of 25 mole-%, preferably in excess of 10 mole-%, relative to the total feed, is avoided. A concentration of carbon dioxide of at most 1 mole-%, preferably at most 0.5 mole-%, more preferably at most 0.2 mole-%, relative to the total feed, may be employed. A concentration of carbon dioxide of at least 0.01 mole-%, preferably at least 0.05 mole-%, more preferably at least 0.1 mole-%, relative to the total feed, may be employed. Inert gases, for example nitrogen or argon, may be present in the feed in a concentration of from 30 to 90 mole-%, typically from 40 to 80 mole-%. Suitable saturated hydrocarbons are methane and ethane. If saturated hydrocarbons are present, they may be present in a quantity of up to 80 mole-%, relative to the total feed, in particular up to 75 mole-%. Frequently they are present in a quantity of at least 30 mole-%, more frequently at least 40 mole-%. Saturated hydrocarbons may be added to the feed in order to increase the oxygen flammability limit.

The epoxidation process may be carried out using reaction temperatures selected from a wide range. Preferably the reaction temperature is in the range of from 150 to 325 °C, more preferably in the range of from 180 to 300 °C.

The epoxidation process is preferably carried out at a reactor inlet pressure in the range of from 1000 to 3500 kPa. "GHSV" or Gas Hourly Space Velocity is the unit volume of gas at normal temperature and pressure (0 °C, 1 atm, i.e. 101.3 kPa) passing over one unit volume of packed catalyst per hour. Preferably, when the epoxidation process is as a gas phase process involving a packed catalyst bed, the GHSV is in the range of from 1500 to 10000 Nl/(l.h). Preferably, the process is carried out at a work rate in the range of from 0.5 to 10 kmole olefin oxide produced per m³ of catalyst per hour, in particular 0.7 to 8 kmole olefin oxide produced per m³ of catalyst per hour, for example 5 kmole olefin oxide produced per m³ of catalyst per hour. As used herein, the work rate is the amount of the olefin oxide produced per unit volume of catalyst per hour and the selectivity is the molar quantity of the olefin oxide formed relative to the molar quantity of the olefin converted.

The olefin oxide produced may be recovered from the reaction mixture by using methods known in the art, for example by absorbing the olefin oxide from a reactor outlet stream in water and optionally recovering the olefin oxide from the aqueous solution by distillation. At least a portion of the aqueous solution containing the olefin oxide may be applied in a subsequent process for converting the olefin oxide into a 1,2-diol or a 1,2-diol ether.

The olefin oxide produced in the epoxidation process may be converted into a 1,2-diol, a 1,2-diol ether, or an alkanolamine.

The conversion into the 1,2-diol or the 1,2-diol ether may comprise, for example, reacting the olefin oxide with water, suitably using an acidic or a basic catalyst. For example, for making predominantly the 1,2-diol and less 1,2-diol ether, the olefin oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-diol ethers in the reaction mixture is increased. The 1,2-diol ethers thus produced may be a di-ether, tri-ether, tetra-ether or a subsequent ether. Alternative 1,2-diol ethers may be prepared by converting the olefin oxide with an alcohol, in particular a primary alcohol, such as methanol or ethanol, by replacing at least a portion of the water by the alcohol.

The conversion into the alkanolamine may comprise, for example, reacting the olefin oxide with ammonia. Anhydrous or aqueous ammonia may be used, although anhydrous ammonia is typically used to favour the production of monoalkanolamine. For methods applicable in the conversion of the olefin oxide into the alkanolamine, reference may be made to, for example US-A-4845296.

The 1,2-diol and the 1,2-diol ether may be used in a large variety of industrial applications, for example in the fields of food, beverages, tobacco, cosmetics, thermoplastic polymers, curable resin systems, detergents, heat transfer systems, etc. The alkanolamine may be used, for example, in the treating ("sweetening") of natural gas.

Unless specified otherwise, the low-molecular weight organic compounds mentioned herein, for example the olefins, 1,2-diols, 1,2-diol ethers, alkanolamines and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

Having generally described the invention, a further understanding may be obtained by reference to the following examples, which are provided for purposes of illustration only and are not intended to be limiting unless otherwise specified.

### Examples

### EXAMPLE 1 - Carrier Preparation:

A heat sinterable material of an alpha alumina powder was used to prepare carrier A and carrier B. The alpha alumina powder had the properties shown in Table 1 below.

**Table 1**

| | |
|---|---|
| volume average particle size | 3.0-3.4 microns |
| Average crystallite size | 1.8-2.2 microns |

The mixture contained the ceramic components shown in Table 2 below. The quantities are in parts by weight (pbw).

**Table 2**

| | |
|---|---|
| Alpha alumina | 98.8 (pbw) |
| Zirconia | 1.0 (pbw) |
| Magnesium silicate | 0.2 (pbw) |

The following ingredients were added in parts by weight as indicated in Table 3:

**Table 3**

| | |
|---|---|
| Ceramic components (see Table 2) | 100 (pbw) |
| Crushed walnut shells | 27 (pbw) |
| Polypropylene | 1.5 (pbw) |
| Boric acid | 0.1 (pbw) |
| Petroleum jelly | 5 (pbw) |

Carrier A was prepared from a mixture as described above in Table 3. The crushed walnut shells used were untreated and had volume average particle size of 250 microns and a quantity of 1660 ppmw of potassium (measured as the element), based on the weight of the crushed walnut shell, which represents in this Example the first concentration of potassium. Carrier B was prepared from a mixture as described above in Table 3. The crushed walnut shells were similar to the crushed walnut shells used to prepare carrier A and were treated according to the invention. In particular, deionized water and crushed walnut shells were combined in an open vessel in a weight ratio of 6:1 deionized water to crushed walnut shells. The deionized water was at ambient temperature. The deionized water was allowed to contact the crushed walnut shells for ten minutes. The water was then drained away from the crushed walnut shells and the crushed walnut shells were dried to yield a treated thermally decomposable material. The concentration of potassium in the treated thermally decomposable material was 660 ppmw, based on the weight of the treated crushed walnut shell, which represents in this Example the second concentration of potassium. The second concentration of potassium was 60% less than the first concentration of potassium. The metallic leachable test for the thermally decomposable material was used to determine the potassium concentrations, as described hereinbefore.

The mixtures were then prepared as follows. The ingredients shown in Table 3 ,except for the petroleum jelly, were mixed for 45 seconds and enough water (about 30 parts by weight) was added to give an extrudable mixture. Mixing was continued for a further 4 minutes. A quantity of petroleum jelly, equivalent to 5 parts by weight, was added and mixing was continued for a further 3 minutes. An extruder was used to produce a plurality of hollow cylinders having an outside diameter of 8 mm, an inside diameter of 1 mm, and a length of 8 mm. The cylinders were dried to less than 2 weight percent moisture. The dried cylinders were then heated in a tunnel kiln to a temperature of 1420°C for about four hours. While in the tunnel kiln the thermally decomposable material was thermally decomposed via combustion and the alumina was sintered. The metallic leachable test for the carrier, as described hereinbefore, was used to determine that Carrier A had 127 ppmw potassium and Carrier B had 73 ppmw potassium which is a 43 % reduction in the concentration of potassium in the carrier made using the treated thermally decomposable material compared to the carrier made using the untreated thermally decomposable material. Table 4 describes the properties of the finished carriers A and B.

**TABLE 4**

| Carrier | Surface area (m²/g) | Water absorption (g/g) | Nitric acid Leachables * | | | | | |
|---|---|---|---|---|---|---|---|---|
| | | | Na | K | Ca | Al | Mg | Si |
| A** | 0.75 | 0.487 | 135 | 127 | 569 | 627 | 62 | 1370 |
| B | 0.74 | 0.469 | 133 | 73 | 568 | 616 | 85 | 1391 |

| | | | | | | | | |
|---|---|---|---|---|---|---|---|---|
| *) ppmw relative to the total carrier **) comparative | | | | | | | | |

### EXAMPLE 2 - Preparation of catalysts:

A silver-amine-oxalate stock solution was prepared by the following procedure:
415 g of reagent-grade sodium hydroxide were dissolved in 2340 ml de-ionized water and the temperature was adjusted to 50 °C.

1699 g high purity "Spectropure" silver nitrate was dissolved in 2100 ml de-ionized water and the temperature was adjusted to 50 °C.

The sodium hydroxide solution was added slowly to the silver nitrate solution, with stirring, while maintaining a solution temperature of 50 °C. This mixture was stirred for 15 minutes, then the temperature was lowered to 40 °C.

Water was removed from the precipitate created in the mixing step and the conductivity of the water, which contained sodium and nitrate ions, was measured. An amount of fresh deionized water equal to the amount removed was added back to the silver solution. The solution was stirred for 15 minutes at 40 °C. The process was repeated until the conductivity of the water removed was less than 90 µmho/cm. 1500 ml fresh deionized water was then added.

630 g of high-purity oxalic acid dihydrate were added in
approximately 100 g increments. The temperature was kept at 40 °C and the pH was kept above 7.8.

Water was removed from this mixture to leave a highly concentrated silver-containing slurry. The silver oxalate slurry was cooled to 30 °C.

699 g of 92 weight percent ethylenediamine (8% de-ionized water) was added while maintaining a temperature no greater than 30 °C. The final solution was used as a stock silver impregnation solution for preparing the catalysts.

Carriers A and B, prepared according to Example 1, were used to make silver catalysts, as follows, to form Catalyst A (comparative) and Catalyst B (according to the invention), respectively. Actual silver and cesium loadings have been specified in Table 5, hereinafter. Catalysts A and B also contained 2.8 mmoles rhenium/kg catalyst, 12 mmoles lithium/kg catalyst, and 0.6 mmoles tungsten/kg catalyst.

### Catalyst B (according to the invention):

Catalyst B was prepared in two impregnation steps.
To 122 grams of stock impregnation solution of specific gravity 1.551 g/ml was added 8.3 grams of water, resulting in a solution with a specific gravity of 1.495 g/ml. A vessel containing 30 grams of Carrier B hollow cylinders was evacuated to 20 mm Hg for 1 minute and the impregnation solution was added to Carrier B while under vacuum, then the vacuum was released and the carrier allowed to contact the liquid for 3 minutes. The impregnated Carrier B was then centrifuged at 500 rpm for 2 minutes to remove excess liquid. Impregnated Carrier B were placed in a vibrating shaker and dried in air flowing at a rate of 16.2 Nl/h at 250°C for 5.5 minutes. The resulting dried Catalyst B Precursor contained approximately 16.2 weight percent silver.

A second solution was made by mixing 122.0 grams of silver stock solution of specific gravity 1.551 g/ml with a solution of 0.1608 g of ammonium perrhenate in 2 g of 1:1 (w/w) ethylenediamine/water, 0.0374 g of ammonium metatungstate dissolved in 2 g of 1:1 ammonia/water and 0.2584 g lithium nitrate dissolved in water. Additional water was added to adjust the specific gravity of the solution to 1.495 g/ml. 50 grams of such doped solution was mixed with 0.1573 g of 46.07 weight percent cesium hydroxide solution. This final impregnation solution was used to prepare Catalyst B. A vessel containing 30 grams of the Catalyst B Precursor was evacuated to 20 mm Hg for 1 minute and the final impregnation solution was added while under vacuum, then the vacuum was released and the precursor allowed to contact the liquid for 3 minutes. The impregnated precursor was then centrifuged at 500 rpm for 2 minutes to remove excess liquid. Catalyst B hollow cylinders were placed in a vibrating shaker and dried in air flowing at a rate of 16.2 Nl/h at 250°C for 5.5 minutes.

### Catalyst A (comparative):

Catalyst A was prepared in the same manner as Catalyst B, using 40 grams carrier A. The specific gravity of the impregnation solution in the first impregnation was 1.476 g/ml. The dried Catalyst A Precursor was then impregnated with a second solution which was made by mixing 218.2 grams of silver stock solution of specific gravity 1.564 g/ml with a solution of 0.3132 g of ammonium perrhenate in 2 g of 1:1 (w/w) ethylenediamine/water, 0.0728 g of ammonium metatungstate dissolved in 2 g of 1:1 ammonia/water and 0.5032 g lithium nitrate dissolved in water. Additional water was added to adjust the specific gravity of the solution to 1.476 g/ml. The total water added was 25.8 grams. 61 grams of such doped solution was mixed with 0.1452 g of 46.07 weight percent cesium hydroxide solution. This final impregnation solution was used to prepare Catalyst A.

### EXAMPLE 3 - Testing of catalysts:

The catalysts were used to produce ethylene oxide from ethylene and oxygen. To do this, crushed samples of the catalysts were loaded into a stainless steel U-shaped tube. The tube was immersed in a molten metal bath (heat medium) and the ends were connected to a gas flow system. The weight of catalyst used and the inlet gas flow rate (0.28 Nl/minute) were adjusted to give a gas hourly space velocity of 3300 Nl/(l.h), as calculated for uncrushed catalyst. The inlet gas pressure was 1550 kPa (absolute).

The gas mixture passed through the catalyst bed, in a "once-through" operation, during the entire test run including the start-up, consisted of 30.0 volume percent ethylene, 8.0 volume percent oxygen, 5.0 volume percent carbon dioxide, 57 volume percent nitrogen and 1.0 to 6.0 parts per million by volume (ppmv) ethyl chloride.

The initial reactor temperature was 180 °C, and this was ramped up at a rate of 10 °C per hour to 225 °C and then adjusted so as to achieve a constant ethylene oxide content of 3.1 volume percent in the outlet gas stream at an ethyl chloride concentration of 2.9 ppmv. For Catalyst A (comparative), the experiment was turned off before reaching 100 days of operation. Performance data at this conversion level are usually obtained for initial peak selectivity. Depending upon the catalyst used and the parameters of the olefin epoxidation process, the time required to reach the initial, peak selectivity, that is the highest selectivity reached in the initial stage of the process, may vary. For example, the initial, peak selectivity of a process may be achieved after only 1 or 2 days of operation or may be achieved after as much as, for example, 1 month of operation. Additionally, selectivities corresponding to increasing cumulative ethylene oxide production would also be measured to obtain stability data.

As may be seen from the data in Table 5, an advantage of the present invention is that catalysts made according to this invention exhibit increased initial selectivity at the same ethylene oxide production levels, as compared to comparative catalyst A, even though the temperature was higher. Operating at higher temperatures tends to lower the selectivity of the catalyst. Also, catalysts made according to this invention exhibit improved activity stability, as evidenced by maintaining lower reaction temperatures. The present invention is also expected to exhibit improved selectivity stability.

**TABLE 5**

| Catalyst | Cesium content (mmoles/kg) | Silver Content %wt. | Selectivity Initial (%) | Temperature Initial (°C) | Selectivity @ 70 Days | TEMP @70 Days | Selectivity @ 100 Days | TEMP @ 100 Days |
|---|---|---|---|---|---|---|---|---|
| A **) | 4.0 | 27.5 | 88.5 | 248 | 88.3 | 254 | ***) | ***) |
| B*) | 4.4 | 27.5 | 89.5 | 256 | 88.9 | 256 | 88.2 | 259 |

| | | | | | | | | |
|---|---|---|---|---|---|---|---|---|
| *) invention **) comparative ***) not tested | | | | | | | | |

## Claims

1. A process for preparing a catalyst comprising depositing a metal on a carrier prepared by:
- treating one or more thermally decomposable materials in particulate form comprising potassium to reduce the total quantity of potassium in the thermally decomposable material by contacting the thermally decomposable material with a fluid,
- preparing a mixture comprising the treated thermally decomposable material and a heat sinterable material selected from alumina, silica, titania, silicon carbide, silicon nitride, niobia, magnesia, zinc oxide, zirconia based compounds, or combinations thereof, and
- heating the mixture to sinter the heat sinterable material and to thermally decompose the treated thermally decomposable material to form the carrier, wherein the thermally decomposable material is an organic material that has a decomposition temperature which is no greater than the sintering temperature of the heat sinterable material, and comprises an organic material and has a volume average particle size of at least 1 micron and at most 1000 microns as measured by a laser light scattering instrument, wherein the total quantity of potassium is reduced by at least 30 percent, calculated based on the weight of the potassium in the treated thermally decomposable material relative to the weight of potassium in the untreated thermally decomposable material.

2. A process as claimed in claim 1, wherein the total quantity of potassium is reduced by at least 40 percent, calculated based on the weight of the potassium in the treated thermally decomposable material relative to the weight of potassium in the untreated thermally decomposable material.

3. A process as claimed in claim 1 or 2, wherein the fluid comprises water.

4. A process as claimed in claim 1 or 2, wherein the treating step comprises a first treatment step comprising contacting the thermally decomposable material with a first treatment fluid, and a second treatment step comprising contacting the thermally decomposable material with a second treatment fluid.

5. A process as claimed in any of claims 1-4, wherein the thermally decomposable material comprises a naturally occurring organic material selected from pecan shells, almond shells, cashew shells, walnut shells, olive pits, apricot pits, peach pits, and corn cobs.

6. A process as claimed in any of claims 1-5, wherein the heat sinterable material comprises alpha alumina.

7. A process as claimed in any of claims 1-6, wherein the metal comprises a catalytically active metallic species selected from silver, molybdenum, nickel, tungsten, and mixtures thereof.

8. A process as claimed in claim 7, wherein the catalytically active metallic species comprises silver.

9. A process as claimed in any of claims 1-8, further comprising depositing a promoter component selected from rhenium, tungsten, molybdenum, chromium, and mixtures thereof.

10. A process as claimed in claim 9, wherein the promoter component comprises rhenium.

11. A process as claimed in claim 10, wherein the promoter component further comprises a rhenium copromoter selected from tungsten, chromium, molybdenum, sulfur, and mixtures thereof.

## Patentansprüche

1. Verfahren zur Herstellung eines Katalysators, umfassend das Abscheiden eines Metalls auf einem Träger, hergestellt durch:
- Behandeln eines oder mehrerer thermisch zersetzbarer Materialien in teilchenförmiger Form, umfassend Kalium, um die Gesamtmenge an Kalium in dem thermisch zersetzbaren Material durch Inkontaktbringen des thermisch zersetzbaren Materials mit einer Flüssigkeit zu verringern,
- Herstellen eines Gemisches, umfassend das behandelte thermisch zersetzbare Material und ein wärmesinterbares Material, ausgewählt aus Aluminiumoxid, Siliciumdioxid, Titandioxid, Siliciumcarbid, Siliciumnitrid, Niob, Magnesia, Zinkoxid, Verbindungen auf Zirkonoxidbasis oder Kombinationen davon und
- Erhitzen des Gemisches zum Sintern des wärmesinterbaren Materials und zum thermischen Zersetzen des behandelten thermisch zersetzbaren Materials unter Bildung des Trägers, wobei das thermisch zersetzbare Material ein organisches Material ist, das eine Zersetzungstemperatur aufweist, die nicht höher als die Sintertemperatur des wärmesinterbaren Materials ist und ein organisches Material umfasst und eine volumenmittlere Teilchengröße von mindestens 1 Mikron und höchstens 1.000 Mikron aufweist, gemessen durch ein Laserlichtstreuungsinstrument, wobei die Gesamtmenge an Kalium um mindestens 30 Prozent reduziert wird, berechnet auf der Grundlage des Gewichts des Kaliums in dem behandelten thermisch zersetzbaren Material im Verhältnis zum Gewicht des Kaliums in dem unbehandelten thermisch zersetzbaren Material.

2. Verfahren nach Anspruch 1, wobei die Gesamtmenge an Kalium um mindestens 40 Prozent reduziert wird, berechnet auf der Grundlage des Gewichts des Kaliums in dem behandelten thermisch zersetzbaren Material im Verhältnis zum Gewicht des Kaliums in dem unbehandelten thermisch zersetzbaren Material.

3. Verfahren nach Anspruch 1 oder 2, wobei das Fluid Wasser umfasst.

4. Verfahren nach Anspruch 1 oder 2, wobei der Behandlungsschritt einen ersten Behandlungsschritt umfasst, der das Inkontaktbringen des thermisch zersetzbaren Materials mit einer ersten Behandlungsflüssigkeit umfasst, und einen zweiten Behandlungsschritt umfasst, der das Inkontaktbringen des thermisch zersetzbaren Materials mit einer zweiten Behandlungsflüssigkeit umfasst.

5. Verfahren nach einem der Ansprüche 1 bis 4, wobei das thermisch zersetzbare Material ein natürlich vorkommendes organisches Material umfasst, ausgewählt aus Pekannussschalen, Mandelschalen, Cashewschalen, Walnussschalen, Olivenkernen, Aprikosenkernen, Pfirsichkernen und Maiskolben.

6. Verfahren nach einem der Ansprüche 1 bis 5, wobei das wärmesinterbare Material Alpha-Aluminiumoxid umfasst.

7. Verfahren nach einem der Ansprüche 1 bis 6, wobei das Metall eine katalytisch aktive Metallspezies umfasst, ausgewählt aus Silber, Molybdän, Nickel, Wolfram und Gemischen davon.

8. Verfahren nach Anspruch 7, wobei die katalytisch aktive Metallspezies Silber umfasst.

9. Verfahren nach einem der Ansprüche 1 bis 8, ferner umfassend das Abscheiden einer Promotorkomponente, ausgewählt aus Rhenium, Wolfram, Molybdän, Chrom und Gemischen davon.

10. Verfahren nach Anspruch 9, wobei die Promotorkomponente Rhenium umfasst.

11. Verfahren nach Anspruch 10, wobei die Promotorkomponente ferner einen Rhenium-Copromotor umfasst, der aus Wolfram, Chrom, Molybdän, Schwefel und Gemischen davon ausgewählt ist.

## Revendications

1. Procédé de préparation d'un catalyseur comprenant le dépôt d'un métal sur un support préparé par :
- le traitement d'un ou de plusieurs matériaux thermiquement décomposables sous forme particulaire comprenant du potassium afin de réduire la quantité totale de potassium dans le matériau thermiquement décomposable en mettant en contact le matériau thermiquement décomposable avec un fluide,
- la préparation d'un mélange comprenant le matériau thermiquement décomposable traité et un matériau frittable à chaud choisi parmi l'alumine, la silice, l'oxyde de titane, le carbure de silicium, le nitrure de silicium, l'oxyde de niobium, l'oxyde de magnésium, l'oxyde de zinc, les composés à base de zircone, ou leurs combinaisons, et
- le chauffage du mélange afin de fritter le matériau frittable à chaud et de décomposer thermiquement le matériau thermiquement décomposable traité pour former le support, le matériau thermiquement décomposable étant un matériau organique qui a une température de décomposition qui n'est pas supérieure à la température de frittage du matériau frittable à chaud, et qui comprend un matériau organique et présente une taille moyenne de particules en volume d'au moins 1 micron et d'au plus 1000 microns telle que mesurée par un instrument de diffusion de lumière laser, la quantité totale de potassium étant réduite d'au moins 30 %, calculée sur la base du poids du potassium dans le matériau thermiquement décomposable par rapport au poids de potassium dans le matériau thermiquement décomposable non traité.

2. Procédé selon la revendication 1, dans lequel la quantité totale de potassium est réduite d'au moins 40 %, calculée sur la base du poids du potassium dans le matériau thermiquement décomposable par rapport au poids du potassium dans le matériau thermiquement décomposable non traité.

3. Procédé selon la revendication 1 ou la revendication 2, dans lequel le fluide comprend de l'eau.

4. Procédé selon la revendication 1 ou la revendication 2, dans lequel l'étape de traitement comprend une première étape de traitement comprenant la mise en contact du matériau thermiquement décomposable avec un premier fluide de traitement, et une seconde étape de traitement comprenant la mise en contact du matériau thermiquement décomposable avec un second fluide de traitement.

5. Procédé selon l'une quelconque des revendications 1 à 4, dans lequel le matériau thermiquement décomposable comprend un matériau organique d'origine naturelle choisi parmi les coques de noix de pécan, les coques d'amandes, les coques de noix de cajou, les coques de noix, les noyaux d'olives, les noyaux d'abricots, les noyaux de pêches et les épis de maïs.

6. Procédé selon l'une quelconque des revendications 1 à 5, dans lequel le matériau frittable à chaud comprend de l'alumine alpha.

7. Procédé selon l'une quelconque des revendications 1 à 6, dans lequel le métal comprend une espèce métallique catalytiquement active choisie parmi l'argent, le molybdène, le nickel, le tungstène et leurs mélanges.

8. Procédé selon la revendication 7, dans lequel l'espèce métallique catalytiquement active comprend de l'argent.

9. Procédé selon l'une quelconque des revendications 1 à 8, comprenant en outre le dépôt d'un composant promoteur choisi parmi le rhénium, le tungstène, le molybdène, le chrome et leurs mélanges.

10. Procédé selon la revendication 9, dans lequel le composant promoteur comprend du rhénium.

11. Procédé selon la revendication 10, dans lequel le composant promoteur comprend en outre un copromoteur de rhénium choisi parmi le tungstène, le chrome, le molybdène, le soufre et leurs mélanges.
